# DEMANDE DE BREVET EUROPEEN

(11) **EP 1 625 831 A1**
(43) Date de publication de la demande: **15.02.2006**
(21) Numéro de dépôt: 05370016.7
(22) Date de dépôt: 29.06.2005
(51) Int. Cl.: A61F 2/00

(54) **Implant prothetique obturateur**

(30) Priorité: 29.06.2004 FR 0407171
(71) Demandeur: Textile Hi Tec, 81270 Labastide Rouairoux (FR)
(72) Inventeur: Houard, William, 81270 Labastide-Rouairoux (FR)
(74) Mandataire: Hennion, Jean-Claude

(57) **Abrégé**

L'implant prothétique obturateur (1) est destiné à être placé , par voie laparotomique, notamment à l'aide d'un tube introducteur. Il est constitué par un empilement d'au moins une première plaque prothétique (2) dans un matériau textile poreux et d'au moins une seconde plaque prothétique (3) dans un matériau composite formé d'un matériau textile poreux dont la face extérieure est enduite d'un matériau non adhérent, toutes les plaques (2,3) de l'empilement étant reliées les unes aux autres au niveau d'une zone centrale (5). La ou les premières plaques sont destinées à être placées en situation transpariétale et la ou les secondes plaques en situation prépéritonéale ou intrapéritonéale.

De préférence toutes les plaques prothétiques (2,3) ont une forme circulaire ou polygonale et sont disposées dans l'empilement , avec un axe de symétrie commun (6), autour duquel se trouve la zone centrale (5) de liaison de toutes les plaques (2,3).

## Description

La présente invention concerne le domaine des implants prothétiques. Elle concerne plus particulièrement un implant obturateur utilisé par voie laparotomique, notamment pour le traitement des hernies inguinales et crurales et des éventrations.

Il est connu, pour réparer un défaut de paroi tissulaire ou musculaire, d'insérer dans ledit défaut un implant prothétique dont la configuration est à même d'épouser la forme du défaut dans lequel il est inséré. On obtient donc une occlusion et donc une réparation de ce défaut grâce à l'implant prothétique obturateur.

Différents types d'implants prothétiques obturateurs ont été proposés. Selon un premier type, connu notamment par les documents US.5.147.374 et EP.O.614.65O, l'implant est préformé et sa configuration est généralement conique ou cylindrique. Dans un second type, connu notamment par les documents EP.1.024.764 et EP.898.944, l'implant se présente à plat sous forme de plaque ou d'association de plaques, la mise en forme n'intervenant qu'au moment du positionnement de l'implant dans le défaut de la paroi.

Ces deux types d'implants ont l'un et l'autre leurs inconvénients.

Les implants préformés présentent une trop grande rigidité qui peut entraîner des traumatismes de la paroi. Généralement leur préformage n'est pas totalement adapté à la configuration réelle du défaut. De plus les implants préformés de forme conique possèdent généralement une extrémité pointue, ce qui peut entraîner des irritations et un inconfort du patient, voire des adhérences au niveau de cette extrémité.

Quelque soit le type d'implant, il y a un risque d'expulsion post-opératoire de l'implant dû à la poussée naturelle qui s'exerce vers l'arrière.

Le but de la présente invention est de proposer un implant prothétique obturateur qui pallie les inconvénients précités.

Il s'agit d'un implant prothétique obturateur destiné à être placé, par voie laparotomique, notamment à l'aide d'un tube introducteur.

De manière caractéristique, selon la présente invention, cet implant est constitué par un empilement d'au moins une première plaque prothétique dans un matériau textile poreux et d'au moins une seconde plaque prothétique dans un matériau composite formé d'un matériau textile poreux dont la face extérieure est enduite d'un matériau non adhérent, toutes les plaques de l'empilement étant reliées les unes aux autres au niveau d'une zone centrale. De plus la ou les premières plaques sont destinées à être placées en situation transpariétale et la ou les secondes plaques en situation prépéritonéale ou intrapéritonéale en cas de défaut ou lésion du péritoine.

Ainsi, selon la disposition particulière de l'implant prothétique obturateur de la présente invention, lorsqu'il est mis en place, la ou les secondes plaques en matériau composite se trouvent disposées entre la paroi musculaire et le péritoine, le matériau anti-adhérent étant tourné vers le péritoine, ce qui empêche que ne se forment des adhérences entre le péritoine proprement dit et la paroi musculaire dans la zone du défaut à réparer. Et quand le péritoine est lésé ou fait défaut, le matériau anti-adhérent est positionné dans la cavité intrapéritonéale sans risque d'adhérence. Quant à la ou les premières plaques en matériau textile poreux, le repliement sur elles-mêmes lors de l'introduction dans le défaut leur confère, une fois insérées, une configuration en corolle qui obture lintégralité du volume du défaut. Bien sûr, le nombre de premières plaques est fonction de la taille du défaut.

La ou les secondes plaques prothétiques placées en situation prépéritonéale ou intrapéritonéale, avec une dimension supérieure à l'orifice herniaire, forment une butée anti-retour qui empêche l'expulsion post-opératoire de l'implant.

Pour obtenir le placement de l'implant tel que précisé ci-dessus, à l'aide d'un tube introducteur, il est préférable que la ou les premières plaques prothétiques soient disposés à l'intérieur du tube introducteur tandis que la ou les secondes plaques prothétiques soient disposées extérieurement à l'extrémité du tube introducteur.

Selon une variante de réalisation, toutes les plaques prothétiques ont une forme circulaire ou polygonale et sont disposées dans l'empilement, avec un axe de symétrie commun, autour duquel se trouve la zone centrale de liaison de toutes les plaques.

La liaison entre toutes les plaques de l'empilement peut être réalisée par couture, par exemple une couture circulaire, en croix ou en carré.

Eventuellement la liaison entre toutes les plaques peut être réalisée par collage, thermosoudage ou par ultrasons.

Dans une variante de réalisation, l'implant prothétique obturateur de l'invention comprend trois premières plaques et une ou éventuellement deux secondes plaques.

Dans le cas où l'implant prothétique comporte plusieurs premières plaques, avantageusement lesdites premières plaques sont de dimensions décroissantes depuis la première plaque qui se trouve directement adjacente à une seconde plaque jusqu'à celle qui lui est la plus éloignée.

Dans ce cas particulier, s'agissant de plaques circulaires, de préférence il y a une dégressivité d'environ 1 cm de diamètre par première plaque.

Grâce à cette dégressivité des premières plaques, seule la plaque de plus grande dimension se trouve en contact avec la paroi interne de l'orifice herniaire, ce qui limite les risque de traumatisme de ladite paroi.

Les autres premières plaques permettent de réaliser le remplissage du volume interne de l'orifice et d'apporter une certaine rigidité à l'ensemble.

C'est à travers toutes les premières plaques que se propage la colonisation cellulaire réparatrice.

Dans le cas où l'implant prothétique comporte plusieurs secondes plaques, de préférence il y a une dégressivité de dimension entre les différentes secondes plaques, depuis la seconde plaque qui est immédiatement adjacente à une première plaque jusqu'à celle qui lui est la plus éloignée.

Dans ce cas particulier, s'agissant de secondes plaques de forme circulaire, de préférence la dégressivité du diamètre est de l'ordre de 1 cm d'une plaque à l'autre.

L'intérêt de disposer plusieurs secondes plaques est d'augmenter la raideur de l'ensemble desdites secondes plaques, notamment lorsqu'elles reprennent leur configuration après passage dans l'orifice herniaire après le retrait du tube introducteur. Avec une seule seconde plaque, trop souple, il peut y avoir un risque que ladite seconde plaque se dispose en corolle et ne soit pas apte à empêcher totalement le risque d'expulsion post-opératoire.

Le matériau textile poreux dans lequel est réalisée chaque première plaque et, pour partie, chaque seconde plaque peut avantageusement être en polypropylène ou en polyéthylène téréphtalate. En ce qui concerne chaque seconde plaque, le matériau non-adhérent recouvrant la face extérieure de celle-ci est de préférence en polyuréthanne, silicone ou polytétrafluoréthylène.

De manière à permettre la colonisation cellulaire, le matériau textile poreux peut présenter une structure tissée, non-tissée ou tricotée. Sa structure peut présenter une configuration tridimensionnelle, avec une certaine épaisseur.

Dans une variante de réalisation, le matériau textile poreux diffère selon qu'il s'agit du matériau textile poreux pour la ou les premières plaques ou du matériau textile poreux pour la ou les secondes plaques.

En particulier lorsque le matériau textile poreux est sous forme d'un non-tissé, avantageusement la ou les premières plaques et/ou la ou les secondes plaques présentent des macro-perforations. Cette disposition particulière permet d'améliorer les capacités d'accrochage pour la colonisation tissulaire et permet aussi d'assurer le drainage, c'est-à-dire l'écoulement des sérosités à travers les secondes plaques.

Dans une variante de réalisation, la ou les premières plaques sont dans un matériau résorbable. La résorption progressive des premières plaques a comme particularité d'activer la colonisation cellulaire. On constate que la réparation obtenue a une résistance plus importante que lorsque le matériau des premières plaques n'est pas résorbable.

Eventuellement, la ou les secondes plaques sont également dans un matériau résorbable, y compris le matériau anti-adhérent. Comme matériau anti-adhérent résorbable, on peut utiliser du collagène ou du PLA (acide polylactique).

Dans un exemple préféré de réalisation, l'implant est constitué de trois premières plaques et de deux secondes plaques, le matériau textile poreux des dites secondes plaques étant sous forme bidimensionnelle.

Dans un autre exemple préféré de réalisation , l'implant comporte trois premières plaques et une seule seconde plaque , le matériau textile poreux dans lequel est réalisée ladite seconde plaque ayant une forme tridimensionnelle.

La présente invention sera mieux comprise à la lecture de la description d'exemples de réalisation d'un implant prothétique obturateur pour le traitement des hernies inguinales et crurales et des éventrations, illustrés par le dessin annexé dans lequel :
La figure 1 est une représentation schématique vue de dessus, en plan, d'un implant prothétique obturateur comportant trois premières plaques prothétiques et deux secondes plaques prothétiques, toutes de forme circulaire, reliées centralement par une couture circulaire.
La figure 2 est une vue schématique en coupe transversale de l'implant de la figure 1 selon l'axe II-II.
La figure 3 est une représentation schématique en perspective d'un ensemble d'obturation comprenant un tube introducteur, un poussoir et l'implant de la figure 1 en position d'introduction et
La figure 4 est une représentation schématique en coupe de l'implant prothétique de la figure 1 mis en place dans le corps du patient.

L'implant prothétique obturateur 1 de la présente invention consiste dans un empilement de deux types de plaques prothétiques, à savoir au moins une première plaque 2 qui est dans un matériau textile poreux et au moins une seconde plaque 3 qui est dans un matériau composite, formé d'une part d'un matériau textile poreux et d'autre part d'un matériau anti-adhérent enduit sur la face extérieure du matériau textile poreux. Toutes ces plaques 2, 3 sont reliées entre-elles dans une zone centrale, par un moyen de liaison 5.

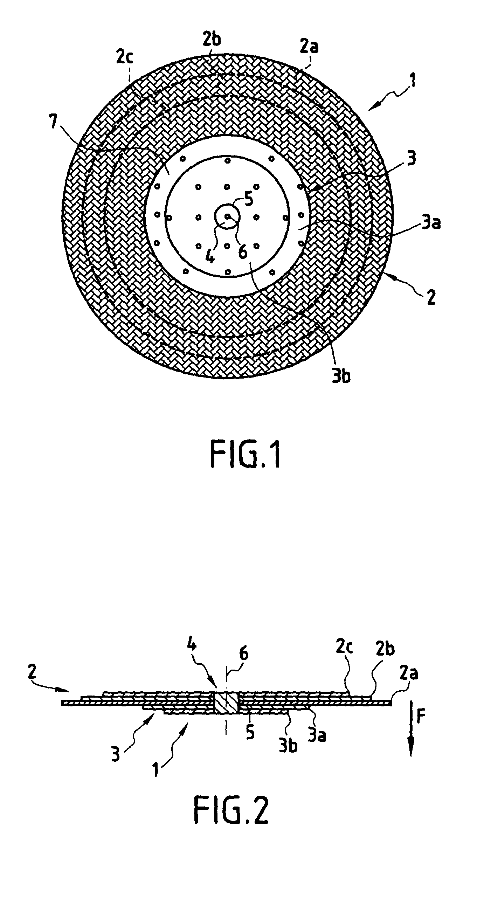

Dans l'exemple illustré à la figure 1, l'implant 1 comporte trois premières plaques 2a, 2b, 2c et deux secondes plaques 3a, 3b. Il s'agit de plaques de forme circulaire, disposées dans l'empilement de manière centrée par rapport à l'axe de symétrie 6. De plus les premières et secondes plaques sont de dimensions différentes les unes des autres.

Comme illustré à la figure 2, on a une décroissance constante du diamètre des premières plaques 2 depuis l'intérieur de l'implant vers l'extérieur, de même une décroissance du diamètre des secondes plaques 3 depuis l'intérieur de l'implant vers l'extérieur. Ainsi si l'on considère le sens d'introduction de l'implant, selon la flèche F, l'empilement des différentes plaques depuis l'avant vers l'arrière est le suivant :
- une seconde plaque 3b ayant un diamètre donné
- une seconde plaque 3a de diamètre supérieur
- une première plaque 2a ayant un diamètre donné
- une première plaque 2b ayant un diamètre inférieur à celui de la première plaque précédente 2a
- une première plaque 2c de diamètre inférieur à celui de la première plaque précédente 2b

Dans un exemple précis de réalisation, donné à titre non-exhaustif, les secondes plaques 3b et 3a ont respectivement 3 et 4 centimètres de diamètre et les trois premières plaques 2a, 2b, 2c ont respectivement 8, 7 et 6 centimètres de diamètre. La dégressivité d'une plaque à l'autre est donc de 1cm.

Dans cet empilement , toutes les plaques sont , dans l'exemple illustré, reliées par une couture circulaire 5 , également centrée sur l'axe de symétrie 6, faisant de l'ordre de 5 à 8 mm.

La face enduite 7 des secondes plaques 3b, 3a est celle qui est tournée vers l'avant de l'implant.

Le nombre de plaques dans l'empilement n'est pas nécessairement de cinq comme dans l'exemple ci-dessus. Il peut dans une variante très simplifiée être de deux, avec une seule première plaque 2 et une seule seconde plaque 3. D'un point de vue pratique, on considère qu'un empilement de dix plaques est un maximum.

Le nombre de deux secondes plaques, comportant extérieurement un matériau anti-adhérent, est préféré lorsque le matériau textile poreux qui supporte ce matériau anti-adhérent est un non-tissé de faible épaisseur.

Par contre lorsque le matériau poreux de cette seconde plaque 3 est un tricot tridimensionnel, une seule seconde plaque est généralement suffisante, sachant que l'épaisseur de cette seconde plaque peut être de l'ordre voire supérieure au millimètre, avec une structure formée d'un réseau tridimensionnel de fils , qui constitue une structure propice à la colonisation tissulaire.

Le matériau textile entrant dans la constitution de la première et de la seconde plaques peut être réalisé à partir de mono-filaments , s'agissant en particulier d'un matériau tissé ou tricoté ou bien à partir de multi filaments , s'agissant en particulier d'un non-tissé. De préférence il est en polypropylène ou en polyéthylène téréphtalate, qui sont des matériaux bien connus dans le domaine des implants chirurgicaux. En ce qui concerne le matériau non-adhérent réalisant l'enduction de la seconde plaque, il peut s'agir de polyuréthanne, de silicone ou de polytétrafluoréthylène.

Le matériau textile poreux constitutif des premières et secondes plaques peut également être résorbable, étant réalisé par exemple dans l'un des matériaux suivants : polylactide, polyglycolide, copolymère polylactide-polyglycolide, caprolactone, polytriméthylcarbonate, polyglactine, dérivé cellulosique, copolymère de ceux-ci ou assemblage de ceux-ci.

Il est à noter que le mode de liaison des différentes plaques dans l'empilement n'est pas obligatoirement une couture , qu'elle soit circulaire comme dans l'exemple illustré , en croix ou carrée. Il peut s'agir d'une liaison par collage, par thermosoudage ou soudage par ultrasons, en fonction du type de matériau textile poreux utilisé.

Dans l'exemple illustré, les deux secondes plaques 3a, 3b sont réalisées sous forme d'un non-tissé enduit d'une couche de polyuréthanne. Pour améliorer leur accrochage avec la paroi musculaire lors de la colonisation tissulaire, ces deux secondes plaques sont pourvues de macro-perforations, faisant de l'ordre ou inférieures à 1 mm, ayant aussi une fonction de drainage.

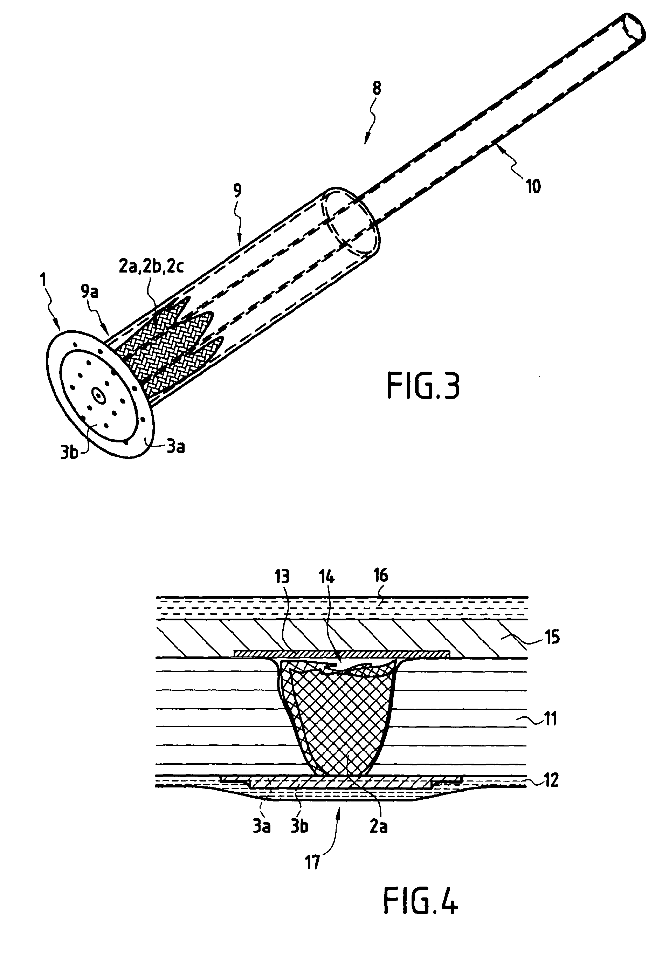

Sur la figure 3 on a représenté un ensemble d'obturation 8 prêt à l'emploi qui comprend un implant 1 décrit ci-dessus, un tube introducteur 9 et un poussoir 10.

Pour son introduction par voie laparotomique, l'implant 1 est disposé au niveau de l'extrémité avant 9a du tube introducteur, avec les premières plaques 2a, 2b et 2c, qui sont repliées en corolle à l'intérieur de ladite extrémité avant tandis que les secondes plaques 3a, 3b sont à l'extérieur du tube introducteur, transversalement à l'axe de celui-ci. Quant au poussoir 10, il pénètre à l'intérieur du tube introducteur, avec son extrémité avant qui se trouve entourée par la corolle de premières plaques 2a, 2b, 2c et qui est centrée sur la zone centrale 5 où se trouve la liaison entre lesdites plaques.

Le praticien introduit le tube introducteur dans l'orifice herniaire jusqu'à insertion des secondes plaques 3a, 3b en situation prépéritonéale ou intrapéritonéale. Il maintient les premières plaques en position dans le tube introducteur, à l'aide du poussoir, tout en effectuant un retrait partiel dudit tube introducteur, en sorte de permettre à la ou aux secondes plaques de se déployer en situation prépéritonéale ou intrapéritonéale. Puis, il retire totalement de l'orifice herniaire le tube introducteur et le poussoir. La ou les secondes plaques forment, dans leur situation prépéritonéale ou intrapéritonéale, une butée qui empêche l'expulsion post-opératoire de l'implant. Les premières plaques qui étaient à l'état replié dans le tube introducteur développent leur corolle dans la partie trans-pariétale pour occuper tout le volume intérieur du défaut.

L'implant 1 ainsi introduit reste bien en place entre la paroi musculaire 11 et le péritoine 12 ou dans la cavité intrapéritonéale 17, sachant bien sûr que les secondes plaques 3a, 3b sont choisies pour avoir un diamètre qui est supérieur à l'orifice herniaire. De plus la colonisation tissulaire et la bonne intégration du matériau textile poreux de la seconde plaque 3a en contact avec la paroi musculaire permet une bonne fixation naturelle de l'implant.

Dans le cas où on utilise pour l'implant des matériaux résorbables, la réaction du corps vis-à-vis de l'implant et de la dégradation des matériaux résorbables entraîne la formation d'une fibrose et donc l'auto-réparation de la paroi musculaire, après disparition totale de l'implant.

Il est possible de mettre en oeuvre une plaque prothétique complémentaire 13 pour recouvrir le défaut 14 après installation de l'implant 1, plaque qui est disposée entre la paroi musculaire 11 et les tissus gras 15 sous la peau 16.

## Revendications

1. Implant prothétique obturateur (1) destiné à être placé , par voie laparotomique , notamment à l'aide d'un tube introducteur, **caractérisé en ce qu'**il est constitué par un empilement d'au moins une première plaque prothétique (2) dans un matériau textile poreux et d'au moins une seconde plaque prothétique (3) dans un matériau composite formé d'un matériau textile poreux dont la face extérieure est enduite d'un matériau non adhérent , toutes les plaques (2,3) de l'empilement étant reliées les unes aux autres au niveau d'une zone centrale (5), et **en ce que** la ou les premières plaques sont destinées à être placées en situation transpariétale et la ou les secondes plaques en situation prépéritonéale ou intrapéritonéale.

2. Implant selon la revendication 1 **caractérisé en ce que** toutes les plaques prothétiques (2,3) ont une forme circulaire ou polygonale et sont disposées dans l'empilement, avec un axe de symétrie commun (6) , autour duquel se trouve la zone centrale (5) de liaison de toutes les plaques (2,3).

3. Implant selon l'une des revendications 1 ou 2 **caractérisé en ce que** la liaison entre toutes les plaques (2,3) de l'empilement est réalisée par couture, par exemple une couture circulaire , en croix ou en carré.

4. Implant selon l'une des revendications 1 ou 2 **caractérisé en ce que** la liaison entre toutes les plaques est réalisée par collage, thermosoudage ou par soudage ultrasonique.

5. Implant selon l'une des revendications 1 à 4 **caractérisé en ce qu'**il comprend de deux à cinq plaques, de préférence trois premières plaques (2a, 2b, 2c) et une ou éventuellement deux secondes plaques (3a, 3b).

6. Implant selon la revendication 5 **caractérisé en ce que** les premières plaques (2) sont de dimensions décroissantes depuis la première plaque (2a) qui se trouve directement adjacente à une seconde plaque (3) jusqu'à celle qui lui est la plus éloignée (2c).

7. Implant selon l'une des revendications 5 ou 6 **caractérisé en ce que** les secondes plaques (3a, 3b) sont de dimensions décroissantes depuis la seconde plaque (3a) qui est immédiatement adjacente à une première plaque (2a) jusqu'à celle qui lui est la plus éloignée (3b).

8. Implant selon l'une des revendications 6 ou 7 **caractérisé en ce que**, s'agissant de plaques circulaires, la dégressivité de dimension est d'environ 1 cm de diamètre d'une plaque à l'autre.

9. Implant selon l'une des revendications 1 à 8 **caractérisé en ce que** le matériau textile poreux dans lequel est réalisée chaque première plaque (2) et, pour partie , chaque seconde plaque (3) est en polypropylène ou en polyéthylène téréphtalate.

10. Implant selon l'une des revendications 1 à 9 **caractérisé en ce que**, le matériau non-adhérent recouvrant la face extérieure de chaque seconde plaque (3) est en polyuréthanne, silicone ou polytétrafluoréthylène.

11. Implant selon l'une des revendications 1 à 10 **caractérisé en ce que** le matériau textile poreux a une structure tissée, non-tissée ou tricotée , de configuration bidimensionnelle ou tridimensionnelle.

12. Implant selon la revendication 11 **caractérisé en ce que** le matériau textile poreux étant sous forme d'un non-tissé , la ou les premières plaques (2) et/ou la ou les secondes plaques (3) présentent des macro-perforations.

13. Implant selon l'une des revendications 1 à 2 **caractérisé en ce que**, la ou les premières plaques (2) et/ou la ou les secondes plaques sont dans des matériaux résorbables.

14. Ensemble d'obturation, prêt à l'emploi, comprenant un tube introducteur 9, un poussoir 10 et un implant selon l'une des revendications 1 à 13, **caractérisé en ce que** la ou les premières plaques prothétiques (2) sont disposées à l'intérieur du tube introducteur (9) tandis que la ou les secondes plaques prothétiques (3) sont disposées extérieurement à l'extrémité du tube introducteur , transversalement à la direction de celui-ci , l'extrémité avant du poussoir étant entourée par les premières plaques (2) repliées et centrée sur la zone centrale (5) de l'implant (1).
